# EUROPÄISCHE PATENTSCHRIFT

(11) **EP 2 277 496 B1**
(45) Veröffentlichungstag und Bekanntmachung des Hinweises auf die Patenterteilung: **30.03.2016**
(21) Anmeldenummer: 10189804.7
(22) Anmeldetag: 18.04.2006
(51) Int. Cl.: A61K 6/00, A61K 6/093, A61K 6/083

(54) **Dentalwerkstoff enthaltend oberflächenmodifizierte Füllstoffe**
Dental material containing surface-modified fillers
Matériau dentaire contenant une charge à surface modifiée

(30) Priorität: 27.04.2005 DE 102005019600
(43) Veröffentlichungstag der Anmeldung: 26.01.2011
(62) Teilanmeldung aus: 06008008.2
(73) Patentinhaber: Ivoclar Vivadent AG, 9494 Schaan (LI)
(72) Erfinder: Moszner, Norbert, 9493 Mauren (LI); Klapdohr, Simone, 83022 Rosenheim (DE); Salz, Ulrich, 88131 Lindau (DE); Rheinberger, Volker, 9490 Vaduz (LI); Zimmermann, Jörg, 8046 Zürich (AT)
(74) Vertreter: UEXKÜLL & STOLBERG

(56) Entgegenhaltungen:
- EP-A- 0 333 503
- WO-A-2004/035649
- PAPE P G ET AL: "IMPROVEMENTS IN SILANE COUPLING AGENTS FOR MORE DURABLE BONDING AT THE POLYMER-REINFORCEMENT INTERFACE", ENGINEERING PLASTICS, RAPRA TECHNOLOGY, SHAWBURY, SHREWSBURY, SHROPSHIRE, GB, Bd. 6, Nr. 3, Januar 1993 (1993-01), Seiten 196-207, XP000382226, ISSN: 0952-6900

## Beschreibung

Die vorliegende Erfindung betrifft Dentalwerkstoffe auf der Basis von oberflächenmodifizierten Füllstoffen, die sich besonders als Adhäsiv und Komposite eignen.

Gefüllte Materialien auf der Basis von (Meth)acrylatmonomeren werden in der restaurativen Zahnheilkunde als Füllungs- und Befestigungsmaterialien, Fissurenversiegler, Zemente für die Kieferorthopädie, als Beschichtungsmaterialien und als Adhäsive eingesetzt. Die hierbei verwendeten Füllstoffe lassen sich in organische und anorganische Füllstoffe einteilen, wobei meistens anorganische Füllstoffe zum Einsatz kommen. Diese können wiederum in oxidische und nicht oxidische Füllstoffe unterteilt werden. Die oxidischen Füllstoffe klassifiziert man dann nochmals in silikatische und nichtsilikatische Füllstoffe.

Zu den silikatischen Füllstoffen gehören beispielsweise gemahlene Gläser, wie z.B. Barium-Silikatgläser (US 4,220,582), Strontium-Silikatgläser (DE 43 23 143) und röntgenopaque Aluminium-Fluorö-Silikatgläser, die vor allem in (meth)acrylatverstärkten Glasionomeren Verwendung finden (US 5,367,002, US 5,871,360).

Zur Verbesserung der mechanischen Eigenschaften werden die Oberflächen der Füllstoffe in der Regel so modifiziert, daß diese bei der Aushärtung des Materials durch Copolymerisation kovalent in die Polymermatrix eingebunden werden. Bei anorganischen, silikatischen Füllstoffen wird meist eine Silanisierung, d.h. eine Oberflächenmodifizierung mit vorhydrolysierten (Meth)acryloxyalkyltrialkoxysilanen, wie z.B. 3-Methacryloxypropyltrimethoxysilan, durchgeführt. Bei der Silanisierung re-, agiert das gebildete Silanol mit freien Si-OH-Gruppen der Füllstoffoberfläche (vgl. E. P. Plueddemann, "Silane Coupling Agents", Plenum Press, 2^{nd} Ed., New York und London, 1991).

Die GB 1 488 403 offenbart Lithium-Aluminium-Silikat-Gläser, die mit Trimethoxy-(3-methacryloyloxypropyl)silan modifiziert werden.

Die DE 40 29 230 offenbart Füllungs- und Befestigungsmaterialien auf der Basis von Mischoxiden z.B. von Zirkonoxid und Siliciumdioxid, die mit α-Methacryloxypropyltrimethoxysilan silanisiert sein können.

Aus der US 2002/0072551 sind Fissurenversiegler bekannt, die unterschiedliche Füllstoffe, wie z.B. gemahlenes Barium- oder Lithiumaluminiumsilikatglas enthalten, das mit Silanen wie γ-Methacryloxypropyltrimethoxysilan, Dimethyldichlorsilan oder Hexamethylendisilazan oberflächenbehandelt ist.

Die US 2002/0065337 schlägt Silane wie 3-Methacryloxypropyltrimethoxysilan, 3-Methacryloxypropyldimethoxymonochlorsilan und 3-Methacryloxypropyldichlormonomethoxysilan etc. zur Silanisierung von Nanopartikeln auf der Basis von gemahlenem Glas, Kieselsäuren, Zeolithen usw. vor.

Aus der US 2004/0010055 sind Glasionomerfüllstoffe bekannt, die mit einem Silan modifiziert sind, das eine Polyalkoxyethylengruppe enthält.

Die DE 24 05 578 A1 offenbart Dentalmaterialien, die amorphe Kieselsäure mit einer maximalen Teilchengröße von 0,07 µm als Füllstoff enthalten. Der Füllstoff wird mit Trimethoxy-(3-methacryloyloxypropyl)silan behandelt.

Aus der DE 32 47 800 A1 sind amorphe, kugelförmige Mischoxide auf Basis von Silicium- und Zirkonoxid bekannt, die mit γ-Methacryloxypropyltrimethoxysilan behandelt werden und sich als Füllstoff für Zahnersatzmaterial eignen sollen.

Die DE 195 08 586 A1 offenbart polymerisierbare Dentalwerkstoffe, die als Füllstoffe kugelförmige Partikel auf der Basis von SiO₂ und Oxiden von Elementen der Gruppen I, II, III und IV des Periodensystems enthalten. Die Füllstoffpartikel können mit einer Schicht aus einem polymerisierbaren organischen Bindemittel überzogen sein. Zur Oberflächenmodifizierung werden die Partikel beispielsweise mit Trimethoxy- oder Triethoxyvinylsilan behandelt. Derartige Mischoxide werden neben der verstärkenden Wirkung zur Erhöhung der Röntgenopazität, zur Einstellung der Transparenz und zur Anpassung des Brechungsindex eingesetzt.

Aus der WO 00/69392 sind nichtsilikatische Füllstoffe auf der Basis von Zirkonoxid bekannt, die mit organofunktionellen Kopplungsmitteln wie hydrolysierbaren Methacryloyloxyzirkonaten oder Methacryloyloxyaluminozirkonaten oberflächenmodifiziert sind. Alternativ können die Kopplungsmittel über Phosphonatbindungen an die Partikeloberfläche gebunden sein. Außerdem ist eine weitere Modifizierung der Partikel mit Silanen wie Dimethylethoxyvinylsilan möglich.

Aus der WO 98/13008 ist oberflächenmodifiziertes Tantaloxid und aus der DE 100 18 405 Yttriumoxid bekannt. Diese Füllstoffe eignen sich besonders als Röntgenkontrastmittel. Aluminium- und Titanoxid dienen demgegenüber wegen ihres hohen Brechungsindex häufig als Trübungsmittel.

Nichtsilikatische Füllstoffe wie Zirkonoxid können gemäß US 6,387,981 auch durch methacrylatmodifizierte Polyethercarbonsäuren oberflächenkonditioniert werden.

Außerdem ist aus der US 6,417,244 die Oberflächenmodifizierung von nichtsilikatischen Füllstoffen mit Methacryloyloxyphosphaten bekannt.

Gemeinsam an den oben beschriebenen Füllstoffen ist, daß zur Oberflächenmodifizierung meist Haftvermittler verwendet werden, die unter sauren Bedingungen nicht stabil sind. In der Zahnheilkunde kommen jedoch immer häufiger selbstätzende, selbstkonditionierende Restaurationsmaterialien zur Anwendung, die sich dadurch auszeichnen, daß keine Präkonditionierung der Zahnhartsubstanz notwendig ist. Dazu zählen die selbstätzenden Dentin/Schmelz-Adhäsive, methacrylatverstärkte Glasionomere, selbsthaftende Komposite oder auch Compomere.

Selbstätzende Dentin/Schmelz-Adhäsive sind meist so aufgebaut, daß sie ein acides Haftmonomer, ein oder mehrere nicht saure Comonomere, Wasser oder wasserhaltige Lösungsmittelgemische, einen Polymerisationsinitiator und gegebenenfalls weitere Additive enthalten.

Die US 6,214,101 offenbart selbstätzende methacrylatverstärkte Glasionomere, die als Paste/Paste-System vorliegen. Eine der Pasten ist eine auf Wasser basierende Mischung, die eine Polysäure, wie z.B. Polyacryl- oder Polymaleinsäure, und silanmodifiziertes Bariumglaspulver enthält.

Werden in derartige selbstätzende Systeme oberflächenmodifizierte Füllstoffe der oben beschriebenen Art eingearbeitet, besteht das Problem, daß die polymerisierbaren Gruppen der Haftvermittler durch Hydrolyse abgespalten werden, so daß eine kovalente Einbindung des Füllstoffs in das Polymernetzwerk nicht mehr möglich ist. Dadurch kommt es zu einer Beeinträchtigung der verstärkenden Wirkung des Füllstoffs, und es tritt eine deutliche Reduktion der mechanischen Eigenschaften des ausgehärteten Dentalmaterials bei Wasserlagerung auf. Durch die Hydrolyse verliert der Dentalwerkstoff mit der Zeit seine klinische Tauglichkeit.

EP0333503 offenbart hydrolysestabile oberflächenmodifizierte Füllstoffe für Dentalwerkstoffe auf der Basis von Acrylatmonomeren mit Phosphorsäure- und Phosphonsäuregruppen. Es werden vernetzende und acide Monomere eingesetzt, sowie Initiatoren.

WO2004/035649 offenbart oberflächenmodifizierte Nanofüllstoffe für Dentalmaterialien, die radikalisch polymerisierbare Gruppen enthalten.

Der Erfindung liegt die Aufgabe zugrunde, Dentalwerkstoffe bereitzustellen, die gegenüber wäßrigen Säuren hydrolysestabil sind.

Die Aufgabe wird erfindungsgemäß durch Dentalwerkstoffe gemäss Anspruch 1 gelöst, die Füllstoff enthalten, der oberflächlich mit einer Verbindung der Formel (I) modifiziert ist,

[(PG)-R¹-Z]ₙ-SP-[Y-R²-(AG)]ₘ (I)

in der
- AG: -P(=O)(OH)₂, -O-P(=O)(OH)₂, vorzugsweise -COOH, -SO₂OH oder eine chelatisierende Gruppe ist,
- R¹: eine C₁-C₃-Alkylengruppe oder Cyclopropylengruppe ist oder entfällt,
- R²: eine C₁-C₁₀-Alkylengruppe ist oder entfällt,
- Y: O, S, CO-NH, O-CO-NH, NH-CO-NH ist oder entfällt, wobei Y entfällt wenn R² entfällt,
- Z: 0, S, CO-NR⁶, O-CO-NH, NH-CO-NH ist oder entfällt, wobei R⁶ H oder C₁-C₆-Alkyl ist,
- PG: eine radikalisch polymerisierbare Gruppe der Formel 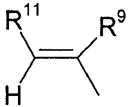 ist, in der R⁹ H, C₁-C₃-Alkyl, C₁-C₃-Hydroxyalkyl oder COOR¹⁰ ist, R¹⁰ H, C₁-C₁₀-Alkyl, 1,6-Dimethylphenyl oder Mesityl ist, R¹¹ H oder Phenyl ist,
- m: 1 oder 2 ist,
- n: 1, 2, 3 oder 4 ist,
- SP: entfällt oder ein (n+m)-wertiger linearer oder verzweigter aliphatischer C₁-C₃₀-Rest, bei dem die Kohlenstoffkette durch O, S, CO-NH, O-CO-NH oder NH-CO-NH unterbrochen sein kann, ein (n+m)-wertiger aromatischer C₆-C₁₈-Rest, ein (n+m)-wertiger cycloaliphatischer C₃-C₁₈-Rest oder ein (n+m)-wertiger heterocyclischer C₃-C₁₈-Rest, ist, wobei die Reste substituiert oder unsubstituiert sein können.

Die Angabe, daß ein Rest durch Fremdatome oder Gruppen, wie Sauerstoff oder Schwefel, unterbrochen sein kann, ist so zu verstehen, daß eines oder mehrere der Fremdatome oder eine oder mehrere der Gruppen in eine Kohlenstoffkette integriert sind. Daraus folgt, daß die Fremdatome oder Gruppen nicht endständig sein können, d.h. eine Anbindung an Nachbargruppen immer über ein Köhlenstoffatom erfolgt, und daß die Zahl der Fremdatome und Gruppen zwangsläufig kleiner als die Zahl der Kohlenstoffatome sein muß.

Formel (I) erstreckt sich nur auf solche Verbindungen, die mit der chemischen Valenzlehre vereinbar sind.

Die bei dem Rest SP gegebenenfalls vorhandenen Substituenten sind vorzugsweise aus C₁-C₅-Alkyl, Cl, Br und/oder OH ausgewählt.

Bevorzugte chelatisierende Gruppen sind zweizähnige, vierzähnige und sechszähnige Liganden. Bevorzugte zweizähnige Liganden sind ß-Diketogruppen der Formel -R⁷-C(=O)-CH₂-C(=O)-R⁸, in der R⁷ C₁-C₃-Alkylen und R⁸ C₁-C₃-Alkyl ist, Salicylsäuregruppen und Salicylaldehydgruppen, Glycinat, Phenanthrolin, Bipyridyl und Ethylendiamin. Besonders bevorzugte zweizähnige Liganden sind die Acetylacetonatgruppe und die Salicylsäuregruppe:

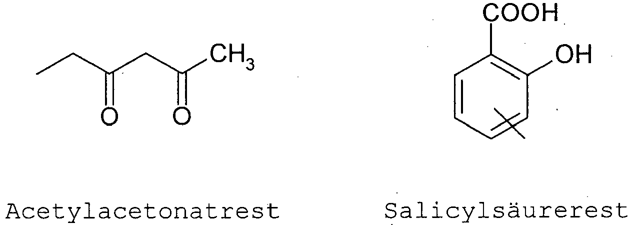

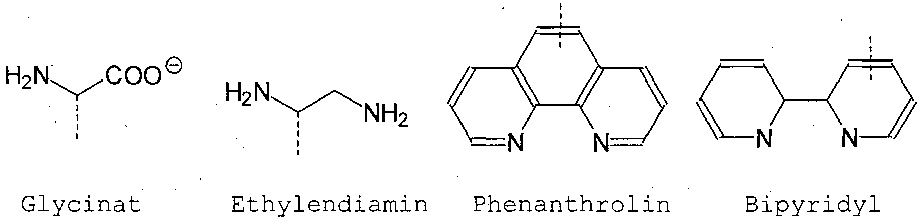

Bevorzugte dreizähnige Liganden sind Diethylentriamin und Iminodiacetat:

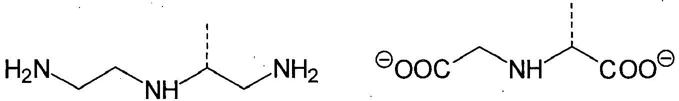

Bevorzugte vierzähnige Liganden sind Tris(2-aminoethyl)amin und Aminotriacetat:

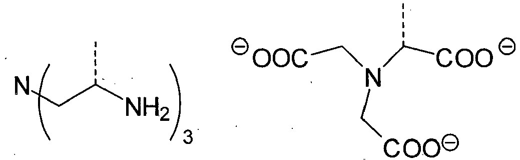

Ein bevorzugter sechszähniger Ligand ist Ethylendiamintetraacetat:

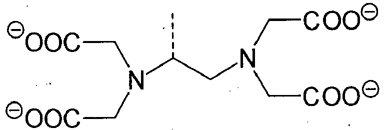

Acide Gruppen sind als Haftgruppen bevorzugt.

PG ist vorzugsweise eine radikalisch polymerisierbare Gruppe der Formel 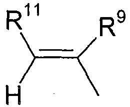 in der
- R⁹: H, CH₃, C₂H₅, Hydroxmethyl, Hydroxyethyl oder COOR¹⁰,
- R¹⁰: H, C₁-C₃-Alkyl, 1, 6-Dimethylphenyl oder Mesityl,
- R¹¹: H oder Phenyl ist.

Bevorzugte polymerisationsfähige Gruppen PG sind Vinylgruppen der Formel H₂C=C(-R⁹)-, Acrylsäuregruppen der Formel H₂C=C(-COOR¹⁰)-, Allyl, Styryl und/oder Vinylcyclopropyl.

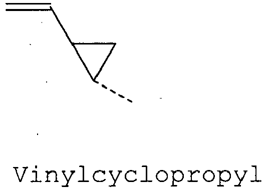

Bevorzugte Definitionen der obigen Variablen, die unabhängig voneinander gewählt werden können, sind:
- AG =: -P(=O)(OH)₂, -O-P(=O)(OH)₂ und insbesondere -COOH,
- R¹ =: eine Methylengruppe, Cyclopropylengruppe oder entfällt,
- R² =: eine C₁-C₃-Alkylengruppe oder entfällt,
- R⁶ =: H, eine C₁-C₃-Alkylgruppe, insbesondere Methyl,
- Z =: CO-NR⁶ oder entfällt,
- Y =: entfällt,
- PG =: für Z = entfällt: eine Vinylgruppe H₂C=C(-R⁹)-, in der R⁹ H, CH₃, C₂H₅, Hydroxmethyl oder Hydroxyethyl ist, oder eine Acrylsäuregruppe H₂C=C(-COOR¹⁰)-, in der R¹⁰ H, C₁-C₃-Alkyl, 1,6-Dimethylphenyl oder Mesityl ist, für Z = CO-NR⁶: eine Vinylgruppe H₂C=C(-R⁹)-, in der R⁹ H oder CH₃ ist,
- m =: 1 oder 2,
- n =: 1 oder 2,
- SP =: entfällt oder ein (n+m)-wertiger linearer oder verzweigter aliphatischer C₁-C₆-Rest, ein (n+m)-wertiger aromatischer C₆-C₁₀-Rest, ein (n+m)-wertiger cycloaliphatischer C₃-C₁₀-Rest oder ein (n+m)-wertiger heterocyclischer C₃-C₁₀-Rest, wobei die Reste substituiert oder unsubstituiert sein können.

Ganz besonders bevorzugt sind Verbindungen, bei mindestens zwei und insbesondere alle Variablen eine der bevorzugten Bedeutungen haben.

Die erfindungsgemäß als Haftvermittler eingesetzten Verbindungen der allgemeinen Formel (I) sind teilweise bekannt und zum Teil sogar kommerziell erhältlich.

Bevorzugte Verbindungen der Formel (I), bei denen AG eine Carboxylgruppe ist (Carbonsäuren), sind α-Hydroxymethylacrylsäure, α,ω-(Meth)acrylamidoalkylencarbonsäuren, (Meth)-acrylamidoarylencarbonsäuren, wie z.B. 3-(Methacryloylamino)-propionsäure, 3-(Acryloylamino)-propionsäure, 6-(Methacryloylamino)-capronsäure, 8-(Methacryloylamino)-caprylsäure, 4-(Methacryloylamino)-benzoesäure oder 4-(Acryloylamino)-benzoesäure:

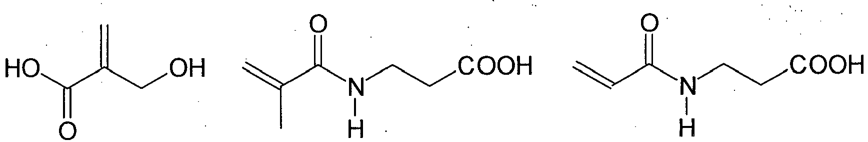

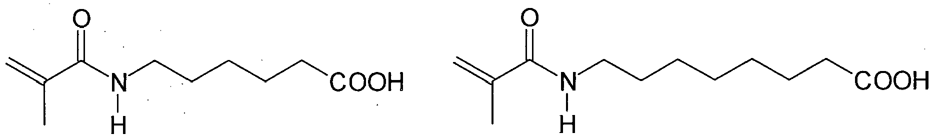

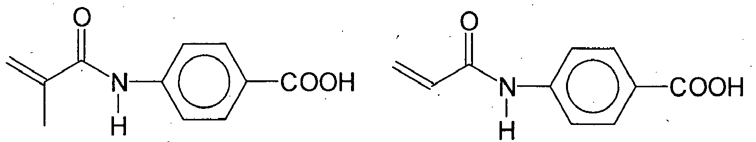

Bevorzugte Verbindungen der Formel (I), bei denen AG eine Phosphonsäuregruppe -P(=)(OH)₂ ist (Phosphonsäuren), sind Vinylphosphonsäure, 4-Vinylbenzylphosphonsäure, und die in EP 909 761 B1 und DE 102 34 326 B3 offenbarten Acrylatetherphosphonsäuren, insbesondere 2-[4-(Dihydroxyphosphoryl)-2-oxa-butyl]-acrylsäureethylester, 2-[4-(Dihydroxyphosphoryl)-2-oxa-butyl]-acrylsäure und 2-[4-(Dihydroxyphosphoryl)-2-oxa-butyl]-acrylsäure-2,4,6-trimethylphenylester. Weiter bevorzugt sind (Meth)acrylamidoalkylenphosphonsäuren und -bisphosphonsäuren, insbesondere 2-Acrylamido- und 2-Methacrylamido-2-methylpropanphosphonsäure (DE 32 10 775), die in DE 33 13 819, JP PS 62 63,314 und Chem. Abstr. 107 (1987), 41318f offenbarten Phosphonsäureverbindungen, insbesondere Methacrylsäure-(2-phosphono-1,1-dimethylethylamin) und die in DD 273 846 A1 beschriebenen N-Acrylaminomethanbisphosphonsäuren:

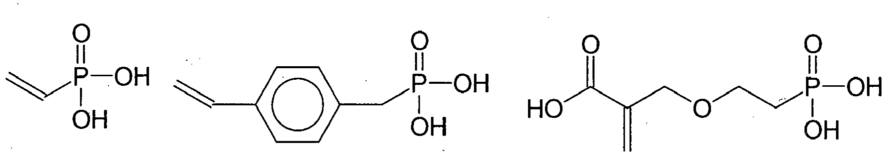

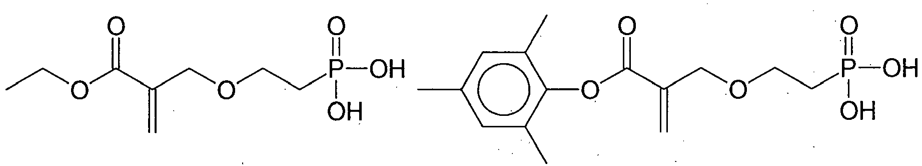

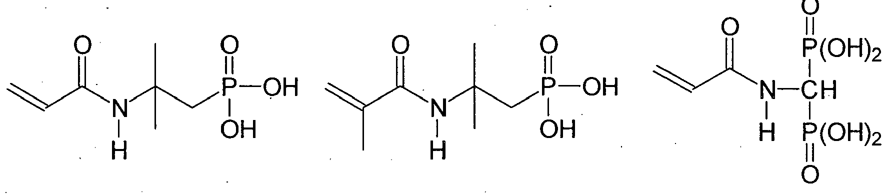

Bevorzugte Verbindungen der Formel (I), bei denen AG eine Phosphorsäuregruppe -O-P(=O)(OH)₂ ist (Dihydrogenphosphate), sind Acryletherphosphate, insbesondere 2-[4-(Dihydroxyphosphoryloxy)-2-oxa-butyl]-acrylsäure und 2-[4-(Dihydroxyphosphoryloxy)-2-oxa-butyl]-acrylsäure-2,4,6-trimethylphenylester, (Meth)acrylamidoalkylen-, -cycloalkylen- und -arylendihydrogenphosphate, insbesondere 2-(N-Acryloylamino)ethyldihydrogenphosphat, 2-(N-Methacryloylamino)ethyldihydrogenphosphat, 6-(N-Acryloylamino)hexyldihydrogenphosphat, 6-(N-Methacryloylamino)hexyldihydrogenphosphat, 4-(N-Acryloylamino)phenyldihydrogenphosphat, 4-(N-Methacryloylamino)phenyldihydrogenphosphat, 1,3-Bis-(N-acryloylamino)-propan-2-yl-dihydrogenphosphat, 1,3-Bis-(N-methacryloylamino)-propan-2-yl-dihydrogenphosphat, 1,3-Bis-(N-acryloyl-N-methyl-amino)-propan-2-yl-dihydrogenphosphat und 1,3-Bis-(N-acryloyl-N-ethyl-amino)-propan-2-yl-dihydrogenphosphat:

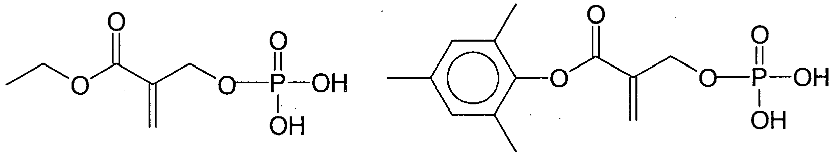

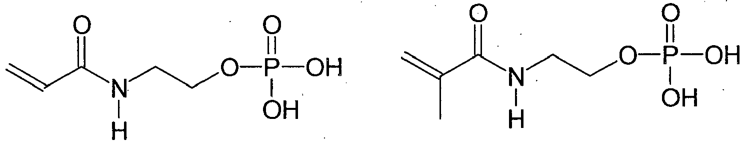

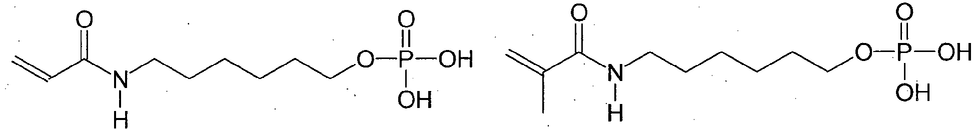

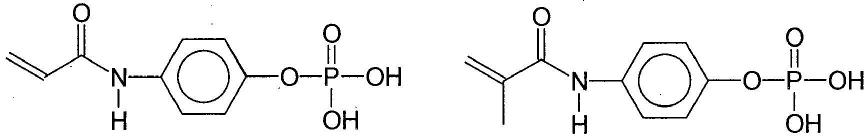

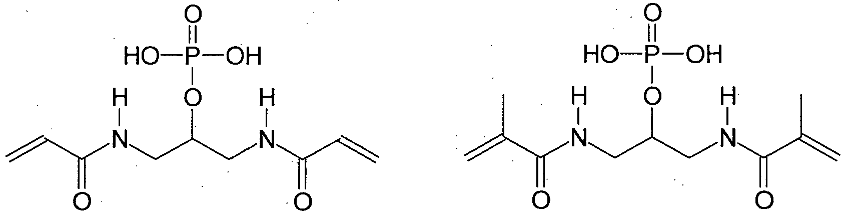

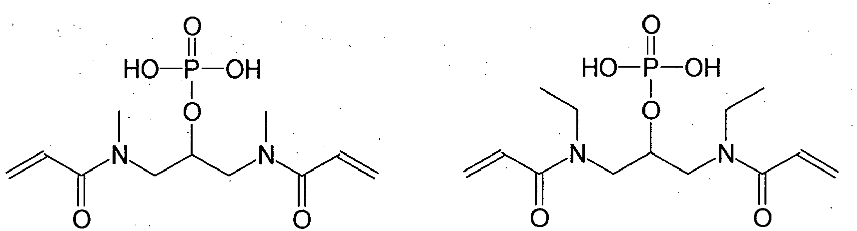

Bevorzugte Verbindungen der Formel (I), bei denen AG eine Sulfonsäuregruppe -SO₂OH ist (Sulfonsäuren), sind Vinylsulfonsäure, 4-Vinylphenylsulfonsäure und 2-Acryloylamino-2-methylpropansülfonsäure:

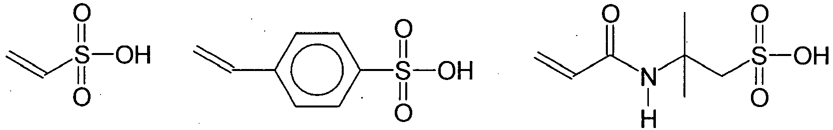

Bevorzugte Verbindungen der Formel (I), bei denen AG eine chelatisierende Gruppe ist, sind Methacryloylaceton (vgl. P. Teyssie, G. Smets, Makromol. Chem. 26 (1958) 245), 4-Vinylbenzoylaceton (A. Mansri, P.F. Casals, A. Oulmidi, K. Guemra, D. Reyx, Eur. Polym. J. 32 (1996) 269) oder 4-Methacryloylaminosalicylsäure:

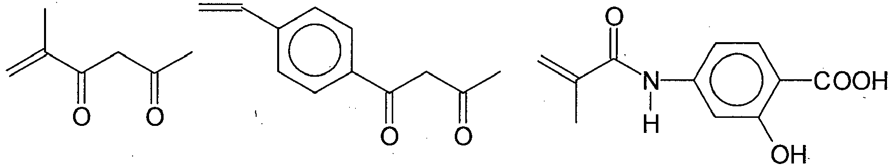

Die Oberflächenmodifizierung der Füllstoffe mit den erfindungsgemäßen Haftvermittlern der Formel (I) kann auf unterschiedlichen Wegen erfolgen.

Flüssige Haftvermittler werden vorzugsweise direkt mit den Füllstoffen vermischt, anschließend werden die Füllstoffe zur Abtrennung von Kondensationsprodukten getrocknet.

Um eine bessere Benetzung der Füllstoffoberfläche zu erreichen, ist es vor allem bei sehr feinteiligen Füllstoffen mit einer spezifischen Oberfläche von mehr als 20 oder 40 m²/g vorteilhaft, den Füllstoff in einer Lösung des Haftvermittlers in einem geeigneten Lösungsmittel zu dispergieren. Dabei läßt sich die Adsorption des Haftvermittlers durch die Füllstoffoberfläche durch die Polarität des Lösungsmittels beeinflussen. Bevorzugte Lösungsmittel sind Cyclohexan, THF, Dioxan, Ethanol und Wasser. Der Grad der Oberflächenmodifizierung hängt dabei unter anderem ab von der Füllstoffmenge und der spezifischen Oberfläche des Füllstoffs, von der Menge des Haftvermittlers, der Reaktionszeit, der Temperatur und von der Füllstoffvorbehandlung, wie z.B. von einer Vortrocknung.

Die verschiedenen Einflußfaktoren sind vor allem bei der Silanisierung von Füllstoffen sehr gut untersucht (vgl. E. P. Plueddemann, "Silane Coupling Agens", Plenum Press, 2nd Ed., New York und London, 1991; A. Guillet, Macromol. Symp. 194 (2003) 63). Bei Silanen erfolgt die Oberflächenmodifizierung durch Hydrolyse der hydrolysierbaren Gruppen am Siliciumatom durch Zugabe von Wasser und einer anschließenden Kondensation z.B. mit Hydroxylgruppen auf der Oberfläche des Füllstoffs. Im Falle von SiO₂ oder silikatischen Füllstoffen bilden sich zwischen Silanol-Gruppen der Füllstoffpartikel und Alkoxysilylgruppen der Silanhaftvermittler Siloxanbindungen aus. Die hydrolysierbaren Gruppen können auch direkt mit den Silanol-Gruppen der Partikeloberfläche unter Bildung einer Siloxanbindung reagieren. Im letzten Fall ist die Zugabe von Wasser nicht erforderlich. Oft reicht auch das Restwasser an der Füllstoffoberfläche aus. Bevorzugt wird dann die Reaktion in unpolareren Lösungsmitteln durchgeführt. Der pH-Wert beeinflußt die Hydrolyse und die Kondensation. Daher wird in der Regel ein saurer oder basischer Katalysator zugegeben. Die saure oder basische Gruppe kann aber auch schon als organischer Rest im Silan enthalten sein oder während der Hydrolyse freigesetzt werden. Um die Reaktion mit der Oberfläche zu beschleunigen kann die Suspension erwärmt werden. Durch anschließendes Verdampfen des Lösungsmittels und Trocknung des Füllstoffs kann die Kondensation weiter forciert werden. Gegebenenfalls können nicht an die Oberfläche gebundene Siloxanspezies in einem anschließenden Waschprozeß vom Füllstoff abgewaschen werden.

Bei Verwendung von Säuren und Chelaten zur Oberflächenmodifizierung werden diese vorzugsweise in einem geeigneten Lösungsmittel, in dem die Säure bzw. der Chelatligand löslich sind, zusammen mit dem dispergierten Füllstoff mehrere Stunden bei Raumtemperatur oder erhöhter Temperatur gerührt. Anschließend wird das Lösungsmittel verdampft und der Füllstoff getrocknet. Alternativ kann der Füllstoff direkt abgetrennt, gewaschen und dann getrocknet werden.

Durch chemische Reaktion der Haftgruppen des Haftvermittlers, z.B. der aciden oder chelatisierenden Gruppen mit geeigneten reaktiven Zentren der Füllstoffoberfläche, wie z.B. OH-Gruppen bei oxidischen Füllstoffen, wie TiO₂ oder Al₂O₃, oder Metallionen von entsprechenden Übergangsmetallverbindungen, z.B. Zr⁴⁺-Ionen bei ZrO₂ oder Yb³⁺-Ionen im Falle von Ytterbiumfluorid, erfolgt eine feste kovalente, ionische oder koordinationschemische Anbindung des Haftvermittlers an die Füllstoffoberfläche.

Nach Abschluß der Reaktion zwischen Füllstoff und Haftvermittler wird der Füllstoff abgetrennt, gegebenenfalls mit demselben oder einem anderen Lösungsmittel gewaschen, einer optionalen Wärmebehandlung unterzogen, gegebenenfalls nochmals gewaschen und dann getrocknet. Dabei kann es bei Füllstoffen, die zur Agglomeratbildung neigen, notwendig sein, diesen nach der Oberflächenmodifizierung zu mahlen oder auf andere Weise zu zerkleinern.

Bevorzugte erfindungsgemäße Füllstoffe sind solche, die auf anorganischen, partikelförmigen Füllstoffen mit einer mittleren Partikelgröße vom 0,01 bis 5 µm basieren. Diese werden erhalten, indem unbehandelte anorganische, partikelförmige Füllstoffe mit einer oder mehreren Verbindungen der Formel (I) auf die oben beschriebene Weise behandelt werden. Unter unbehandelten Füllstoffen werden Füllstoffe verstanden, die noch nicht mit Verbindungen der Formel (I) modifiziert wurden. Diese werden im folgenden auch als Ausgangsmaterialien oder Ausgangsfüllstoffe bezeichnet.

Bevorzugte Ausgangsmaterialien zur Herstellung der erfindungsgemäßen Füllstoffe sind amorpher Füllstoff auf der Basis von einem oder mehreren Metalloxiden und/oder Siliciumoxid, besonders bevorzugt unbehandelte Füllstoffe auf der Basis von ZrO₂, Ta₂O₅, TiO₂, Mischoxiden von SiO₂, ZrO₂ und/oder TiO₂, Yb₂O₃, Y₂O₃, YbF₃, Al₂O₃ und AlO(OH), Böhmit, pyrogener Kieselsäure oder Fällungskieselsäure.

Weiterhin sind Quarz-, Glaskeramik- oder Glaspulver, vorzugsweise mit einer mittleren Partikelgröße von 0,01 bis 5 µm, und röntgenopake Metallverbindungen wie Ytterbiumtrifluorid als Ausgangsfüllstoffe bevorzugt. Wenn nicht anders angegeben handelt es sich bei der mittleren Partikelgröße immer um das Gewichtsmittel, das vorzugsweise durch Lichtstreuung ermittelt wird.

Die Ausgangsfüllstoffe weisen vorzugsweise eine spezifische Oberfläche von mehr als 20 m²/g und insbesondere mehr als 40 m²/g auf, wobei Füllstoffe mit einer maximalen spezifischen Oberfläche von 300 m²/g besonders bevorzugt sind.

Füllstoffe ohne oder mit nur einem geringem SiO₂-Anteil werden vorzugsweise mit solchen Verbindungen der Formel (I) behandelt, bei denen AG -P(=O)(OH)₂ oder -O-P(=O)(OH)₂ ist. Unter einem geringem Si02-Anteil wird ein SiO₂-Gehalt von weniger als 25 Gew.-% und insbesondere weniger als 10 Ges.-% bezogen auf die Masse des jeweiligen Füllstoffs bei gleichmäßiger Verteilung des SiO₂ im Füllstoff verstanden. Bei einer Anreicherung des SiO₂ im Oberflächenbereich des Füllstoffs erniedrigen sich die angegebenen Konzentrationen entsprechend.

Die erfindungsgemäß mit einer Verbindung der Formel (I) modifizierten Füllstoffe eignen sich besonders zur Herstellung von Dentalwerkstoffen. Zur Herstellung von Dentalmaterialien werden die mit Verbindungen der Formel (I) modifizierten Füllstoffe mit einem radikalisch polymerisierbarem Bindemittel und ggf. einem Initiator für die radikalische Polymerisation gemischt.

Zum Bindemittel können hydrolysestabile Verdünnermonomere, wie hydrolysestabile Mono(meth) acrylate, z.B. Mesitylmethacrylat, N-mono- oder N, N-disubstituierte Acrylamide, wie z.B. N-Ethylacrylamid, N,N-Dimethacrylamid, N-(2-Hydroxyethyl)acrylamid und N-Methyl-N-(2-hydroxyethyl)acrylamid, N-monosubstituierte Methacrylamide, wie z.B. N-Ethylmethacrylamid und N-(2-Hydroxyethyl)methacrylamid, sowie N-Vinylpyrrolidon zugegeben werden.

Im Zusammenhang mit der vorliegenden Erfindung werden unter Verdünnermonomeren solche flüssigen Monomeren mit einer oder mehreren polymerisationsfähigen Gruppen verstanden, die sich durch eine Viskosität η von kleiner 100 mPa·s (gemessen bei 20 °C) auszeichnen.

Als hydrolysestabil werden solche Monomere bezeichnet, die in Wasser oder in Mischungen von Wasser und wassermischbaren Lösungsmitteln bei einer Konzentration von ca. 20 Gew.-% und einem pH-Wert von ca. 2,0 bei 37 °C für mindestens 6 Wochen stabil sind, d.h. zu weniger als 5 % hydrolysieren.

Es werden hydrolysestabile Vernetzermonomere als Bindemittel verwendet. Unter Vernetzermonomeren werden Monomere mit zwei oder mehr, vorzugsweise 2 bis 4 radikalisch polymerisierbaren Gruppen verstanden. Bevorzugte Vernetzermonomere sind vernetzende Pyrrolidone, wie z.B. 1,6-Bis(3-vinyl-2-pyrrolidonyl)-hexan, oder kommerziell zugängliche Bisacrylamide, wie Methylen- oder Ethylenbisacrylamid, Bis(meth)-acrylamide, wie z.B. N,N'-Diethyl-1,3-bis(acrylamido)-propan, 1,3-Bis(methacrylamido)-propan, 1,4-Bis(methacrylamido)-butan, 1,4-Bis(acrylamido)-butan und 1,4-Bis(acrylamido)-piperazin, die durch Umsetzung aus den entsprechenden Diaminen mit (Meth)acrylsäurechorid synthetisiert werden können. Die Vernetzermonomere können allein oder zusammen mit einem oder mehreren Verdünnermonomeren als Bindemittel eingesetzt werden.

Das Bindemittel enthält mindestens ein acides Monomer, im folgenden auch als Adhäsivmonomer bezeichnet, das aus 2-[4-(Dihydroxyphosphoryl)-2-oxa-butyl]-acrylsäureethylester, 2-[4-(Dihydroxyphosphoryl)-2-oxa-butyl]-acrylsäure, 2-[4-(Dihydroxyphosphoryl)-2-oxa-butyl]-acrylsäure-2,4,6-trimethylphenylester, 6-(N-Acryloylamino)hexyldihydrogenphosphat, 6-(N-Methacryloylamino)hexyldihydrogenphosphat, 1,3-Bis-(N-acryloylamino)-propan-2-yl-dihydrogenphosphat, und 1,3-Bis-(N-methacryloylamino)-propan-2-yl-dihydrogenphosphat ausgewählt ist.

Die aciden Monomere sind Bestandteil des Bindemittels und können sich darin im Gegensatz zu den zur Oberflächenmodifizierung der Füllstoffe eingesetzten Substanzen frei bewegen. Die an die Füllstoffoberfläche gebundenen Verbindungen weisen keine haftungsvermittelnden Eigenschaften mehr auf.

Als Bindemittel können einzelne Monomere oder Mischungen von zwei oder mehr Monomeren eingesetzt werden, wobei das Bindemittel mindestens ein Vernetzermonomer und Bindemittel, die ausschließlich vernetzende Monomere enthalten, bevorzugt sind, und mindestens ein acides Monomer enthält.

Füllstoff und Bindemittel werden unter Zugabe von einem oder mehreren Lösungsmitteln miteinander gemischt, wobei Aceton, Isopropanol und/oder Ethanol als Lösungsmittel verwendet werden. Anschließend wird der Initiator und gegebenenfalls weitere Additive zugegeben.

Die erfindungsgemäßen Dentalmaterialien lassen sich durch radikalische Polymerisation aushärten. Je nach Wahl des Initiators kann die Härtung durch photochemische oder redoxinduzierte radikalische Polymerisation erfolgen.

Beispiele für bevorzugte Photoinitiatoren sind Benzophenon, Benzoin sowie deren Derivate oder α-Diketone oder deren Derivate wie 9,10-Phenanthrenchinon, Diacetyl oder 4,4-Dichlorbenzil. Besonders bevorzugt werden Campherchinon und 2,2-Dimethoxy-2-phenyl-acetophenon und besonders bevorzugt α-Diketone in Kombination mit Aminen als Reduktionsmittel, wie z.B. 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.

Als Initiatoren für eine bei Raumtemperatur durchgeführte Polymerisation werden Redox-Initiatorkombinationen, vorzugsweise Kombinationen von Benzoylperoxid mit N,N-Dimethylsym.-xylidin oder N,N-Dimethyl-p-toluidin, verwendet. Darüber hinaus sind auch Redoxsysteme bestehend aus Peroxiden und solchen Reduktionsmittel, wie z.B. Ascorbinsäure, Barbiturate oder Sulfinsäuren, besonders geeignet.

Besonders bevorzugt sind Dentalwerkstoffe, welche die folgenden Komponenten enthalten:
(a) 5 bis 90 Gew.-%, insbesondere 5 bis 50 Gew.-% Füllstoff, der mit Haftvermittler der Formel (I) oberflächenmodifiziert ist;
(b) 9,9 bis 90 Gew.-%, insbesondere 0 bis 40 Gew.-%, radikalisch polymerisierbares Monomer (Bindemittel);
(c) 0,1 bis 5,0 Gew.-%, insbesondere 0,2 bis 2,0 Gew.-%, Initiator für die radikalische Polymerisation,
und
(d) bis 70 Gew.-%, insbesondere bis 50 Gew.-%,
Aceton, Isopropanol und/oder Ethanol.

Alle Prozentangaben beziehen sich, wenn nicht anders angegeben, auf die Gesamtmasse des Dentalwerkstoffs.

Der Dentalwerkstoff kann einen oder mehrere Füllstoffe enthalten, die mit einer Verbindung der Formel (I) modifiziert sind. Es ist jedoch bevorzugt, daß alle Füllstoffkomponenten des Dentalwerkstoffs in der angegebenen Weise modifiziert sind.

Acide Monomere werden vorzugsweise in einer Menge von bis zu 70 Gew.-%, besonders bevorzugt bis zu 50 Gew.-% und ganz besonders bevorzugt 3 bis 30 Gew.-% eingesetzt, bezogen auf die Gesamtmasse des Werkstoffs.

Die Dentalwerkstoffe eignen sich besonders als Adhäsive, z.B. Schmelz/Dentin-Adhäsive, selbsthaftende Komposite, Füllungskomposite, Befestigungszemente, Fissurenversiegler oder Beschichtungsmaterialien.

## Patentansprüche

1. Dentalwerkstoff , **dadurch gekennzeichnet, daß** er
a) Füllstoff, der oberflächlich mit einer Verbindung der Formel (I) modifiziert ist,
[(PG)-R¹-Z]ₙ-SP-[Y-R²-(AG)]ₘ (I)
in der
AG -COOH; -P(=O)(OH)₂, -O-P(=O)(OH)₂, -SO₂OH oder eine chelatisierende Gruppe ist,
R¹ eine C₁-C₃-Alkylengruppe oder Cyclopropylengruppe ist oder entfällt,
R² eine C₁-C₁₀-Alkylengruppe ist oder entfällt,
Y O, S, CO-NH, O-CO-NH, NH-CO-NH ist oder entfällt, wobei Y entfällt wenn R² entfällt,
Z O, S, CO-NR⁶, O-CO-NH, NH-CO-NH ist oder entfällt, wobei R⁶ H oder C₁-C₆-Alkyl ist,
PG eine radikalisch polymerisierbare Gruppe der Formel 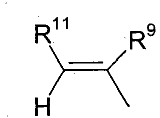 ist, in der R⁹ H, C₁-C₃-Alkyl, C₁-C₃-Hydroxyalkyl oder COOR¹⁰ ist, R¹⁰ H, C₁-C₁₀-Alkyl, 1, 6-Dimethylphenyl oder Mesityl ist, R¹¹ H oder Phenyl ist,
m 1 oder 2 ist,
n 1, 2, 3 oder 4 ist,
SP entfällt oder ein (n+m)-wertiger linearer oder verzweigter aliphatischer C₁-C₃₀-Rest, bei dem die Kohlenstoffkette durch 0, S, CO-NH, O-CO-NH oder NH-CO-NH unterbrochen sein kann, ein (n+m)-wertiger aromatischer C₆-C₁₈-Rest, ein (n+m)-wertiger cycloaliphatischer C₃-C₁₈-Rest.oder ein (n+m)-wertiger heterocyclischer C₃-C₁₈-Rest, wobei die Reste durch C₁-C₅-Alkyl, Cl, Br und/oder OH substituiert oder unsubstituiert sein können,
b) radikalisch polymerisierbares Bindemittel, das
- ggf. mindestens ein hydrolysestabiles Verdünnungsmonomer, welches aus Mono-(meth)acrylaten, N-mono- oder N,N-disubstitiuierten Acrylamiden, N-monosubstituierte Methacrylamiden und N-Vinylpyrrolidonen ausgewählt ist,
- mindestens ein hydrolysestabiles Vernetzermonomer, welches unter vernetzenden Pyrrolidonen, Bisacrylamiden, Bis(meth)-acrylamiden ausgewählt ist, und
- mindestens ein acides Monomer mit mindestens einer Säuregruppe enthält, wobei unter hydrolysestabilen Monomeren solche Monomere verstanden werden, die in Wasser oder in Mischungen von Wasser und wassermischbaren Lösungsmitteln bei einer Konzentration von 20 New.-% und einem pH-Wert von 2,0 bei 37°C innerhalb von 6 Wochen zu weniger als 5 % hydrolysieren, und wobei das acide Monomer aus 2-[4-(Dihydroxyphosphoryl)-2-oxa-butyl]-acrylsäureethylester, 2-[4-(Dihydroxyphosphoryl)-2-oxa-butyl]-acrylsäure, 2-[4-(Dihydroxyphosphoryl)-2-oxa-butyl]-acrylsäure-2,4,6-trimethylphenylester, 6-(N-Acryloylamino)hexyldihydrogenphosphat, 6-(N-Methacryloylamino)hexyldihydrogenphosphat, 1,3-Bis-(N-acryloylamino)-propan-2-yl-dihydrogenphosphat und 1,3-Bis-(N-methacryloylamino)-propan-2-yl-dihydrogenphosphat ausgewählt ist,
c) Initiator für die radikalische Polymerisation und
d) Aceton, Isopropanol und/oder Ethanol enthält.

2. Dentalwerkstoff nach Anspruch 1, bei dem die chelatisierende Gruppe eine ß-Diketogruppe -R⁷-C(=O)-CH₂-C(=O)-R⁸, in der R⁷ C₁-C₃-Alkylen und R⁸ C₁-C₃-Alkyl ist, eine Salicylsäuregruppe, eine Salicylaldehydgruppe, Glycinat, Phenanthrolin, Bipyridyl, Ethylendiamin, Diethylentriamin, Iminodiacetat, Tris(2-aminoethyl)amin und Aminotriacetat oder Ethylendiamintetraacetat ist.

3. Dentalwerkstoff nach einem der vorhergehenden Ansprüche, bei dem PG eine Vinylgruppe der Formel H₂C=C(-R⁹)-, eine Acrylsäuregruppe der Formel H₂C=C(-COOR¹⁰)-, Allyl, Styryl und/oder Vinylcyclopropyl ist.

4. Dentalwerkstoff nach einem der vorhergehenden Ansprüche, bei dem mindestens eine der Variablen der Formel (I) eine der folgenden Bedeutungen hat:
AG = -P(=O)(OH)₂, -O-P (=O)(OH)₂, oder -COOH,
R¹ = eine Methylengruppe, Cyclopropylengruppe oder entfällt,
R² = eine C₁-C₃-Alkylengruppe oder entfällt,
Z = CO-NR⁶ oder entfällt,
Y = entfällt,
PG = für Z = entfällt: eine Vinylgruppe H₂C=C(-R⁹)-, in der R⁹ H, CH₃, C₂H₅, Hydroxymethyl oder Hydroxyethyl ist, oder eine Acrylsäuregruppe H₂C=C(-COOR¹⁰)-, in der R¹⁰ H, C₁-C₃-Alkyl, 1,6-Dimethylphenyl oder Mesityl ist, für Z = CO-NR⁶: eine Vinylgruppe H₂C=C(-R⁹)-, in der R⁹ H oder CH₃ ist,
m = 1 oder 2,
n = 1 oder 2,
SP = entfällt oder ein (n+m)-wertiger linearer oder verzweigter aliphatischer C₁-C₆-Rest, ein (n±m)-wertiger aromatischer C₆-C₁₀-Rest, ein (n+m)-wertiger cycloaliphatischer C₃-C₁₀-Rest oder ein (n+m)-wertiger heterocyclischer C₃-C₁₀-Rest, wobei die Reste substituiert oder unsubstituiert sein können.

5. Dentalwerkstoff nach einem der vorhergehenden Ansprüche, bei dem SP durch C₁-C₅-Alkyl, Cl, Br und/oder OH substituiert ist.

6. Dentalwerkstoff nach einem der Ansprüche 1 bis 5, in dem der Füllstoff ein amorpher Füllstoff auf der Basis von einem oder mehreren Metalloxiden und/oder Siliciumoxid ist.

7. Dentalwerkstoff nach Anspruch 6, in dem der Füllstoff auf ZrO₂, Ta₂O₃, TiO₂, einem Mischoxid von SiO₂, ZrO₂ und/oder TiO₂, Yb₂O₃, Y₂O₃, YbF₃, Al₂O₃ und AlO(OH) Böhmit, pyrogener Kieselsäure oder Fällungskieselsäure basiert.

8. Dentalwerkstoff nach einem der Ansprüche 1 bis 5, in dem der Füllstoff auf Quarz-, Glaskeramik- oder Glaspulver basiert.

9. Dentalwerkstoff nach einem der Ansprüche 1 bis 5, in dem der Füllstoff auf einer röntgenopaken Metallverbindung wie Ytterbiumtrifluorid basiert.

10. Dentalwerkstoff nach einem der vorhergehenden Ansprüche, der
(a) 5 bis 90 Gew.-% Füllstoff, der oberflächlich mit einer Verbindung der Formel (I) modifiziert ist,
(b) 9,9 bis 90 Ges.% radikalisch polymerisierbares Monomer,
(c) 0,1 bis 5,0 Gew.-% Initiator für die radikalische Polymerisation, und
(d) bis 70 Gew.-% Lösungsmittel,
enthält, jeweils bezogen auf die Gesamtmasse des Dentalwerkstoffs.

11. Dentalwerkstoff nach einem der vorhergehenden Ansprüche, der ein Verdünnermonomer, welches aus Mesitylmethacrylat, N-Ethylacrylamid, N,N-Dimethacrylamid, N-(2-Hydroxyethyl)acrylamid, N-Methyl-N-(2-hydroxyethyl)acrylamid, N-Ethylmethacrylamid und N-(2-Hydroxyethyl)-methacrylamid ausgewählt ist, und/oder ein Vernetzermonomer enthält, welches aus 1,6-Bis(3-vinyl-2-pyrrolidonyl)-hexan, Methylen- und Ethylenbisacrylamid, N,N'-Diethyl-1,3-bis(acrylamido)-propan, 1,3-Bis(methacrylamido)-propan, 1,4-Bis(methacrylamido)-butan, 1,4-Bis(acrylamido)-butan und 1,4-Bis(acrylamido)-piperazin ausgewählt ist.

## Claims

1. Dental material, **characterised in that** it comprises
a) filler that is surface-modified with a compound of Formula (I),
[(PG)-R¹-Z]ₙ-SP-[Y-R²-(AG)]ₘ (I)
in which
AG is -COOH, -P(=O)(OH)₂, -O-P(=O)(OH)₂, -SO₂OH or a chelating group,
R¹ is a C₁-C₃ alkylene group or cyclopropylene group or is absent,
R² is a C₁-C₁₀ alkylene group or is absent,
Y is O, S, CO-NH, NH-CO-NH or is absent, wherein Y is absent when R² is absent,
Z is O, S, CO-NR⁶, O-CO-NH, NH-CO-NH or is absent, wherein R⁶ is H or C₁-C₆ alkyl,
PG is a radically polymerisable monomer of the Formula 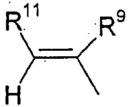 in which R⁹ is H, C₁-C₃ alkyl, C₁-C₃ hydroxyalkyl or COOR¹⁰, R¹⁰ is H, C₁-C₁₀ alkyl, 1,6-dimethylphenyl or mesityl, R¹¹ is H or phenyl,
m is 1 or 2,
n is 1, 2, 3 or 4,
SP is absent or an (n+m)-valent linear or branched aliphatic C₁-C₃₀ residue in which the carbon chain can be interrupted by O, S, CO-NH, O-CO-NH or NH-CO-NH, an (n+m)-valent aromatic C₆-C₁₈ residue, an (n+m)-valent cycloaliphatic C₃-C₁₈ residue or an (n+m)-valent heterocyclic C₃-C₁₈ residue, wherein the residues can be substituted by C₁-C₅ alkyl, Cl, Br and/or OH or can be unsubstituted.
b) radically polymerisable binding agent that comprises
- optionally at least one hydrolysis-stable diluent monomer that is selected from mono(meth)acrylates, N-mono- or *N,N*-disubstituted acrylamides, *N*-mono-substituted methacrylamides and *N*-vinyl pyrrolidones,
- at least one hydrolysis-stable crosslinking monomer that is selected from crosslinking pyrrolidones, bisacrylamides, bis(meth)acrylamides, and
- at least one acidic monomer with at least one acid group, wherein hydrolysis-stable monomers are understood to mean those monomers that hydrolyse to less than 5 % within 6 weeks at 37 °C in water or in mixtures of water and water-miscible solvents at a concentration of 20 wt % and a pH of 2.0, and wherein the acidic monomer is selected from ethyl 2-[4-(dihydroxyphosphoryl)-2-oxa-butyl]acrylate, 2-[4-(dihydroxyphosphoryl)-2-oxa-butyl]acrylic acid, 2,4,6-trimethylphenyl 2-[4-(dihydroxyphosphoryl)-2-oxa-butyl]-acrylate, 6-(*N*-acryloylamino)hexyl dihydrogen phosphate, 6-(*N*-methacryloylamino)hexyl dihydrogen phosphate, 1,3-bis-(*N*-acryloylamino)-propan-2-yl dihydrogen phosphate and 1,3-bis-(*N*-methacryloylamino)-propan-2-yl dihydrogen phosphate,
c) an initiator for the radical polymerisation and
d) acetone, isopropanol and/or ethanol.

2. Dental material according to claim 1, in which the chelating group is a β-diketo group -R⁷-C(=O)-CH₂-C(=O)-R⁸, in which R⁷ is C₁-C₃ alkylene and R⁸ is C₁-C₃ alkyl, a salicylic acid group, a salicylic aldehyde group, glycinate, phenanthroline, bipyridyl, ethylenediamine, diethylenetriamine, iminodiacetate, tris(2-aminoethyl)amine and aminotriacetate or ethylenediaminetetraacetate.

3. Dental material according to one of the preceding claims, in which PG is a vinyl group of the Formula H₂C=C(-R⁹)-, an acrylic acid group of the Formula H₂C=C(-COOR¹⁰)-, allyl, styryl and/or vinylcyclopropyl.

4. Dental material according to one of the preceding claims, in which at least one of the variables of the Formula (I) has one of the following meanings:
AG = ⁻P(=O)(OH)₂, -O-P(=O)(OH)₂, or -COOH,
R¹ = a methylene group, cyclopropylene group or is absent,
R² = a C₁-C₃ alkylene group or is absent,
Z = CO-NR⁶ or is absent,
Y = is absent,
PG = when Z = absent: a vinyl group H₂C=C(-R⁹)-, in which R⁹ is H, CH₃, C₂H₅, hydroxymethyl or hydroxyethyl, or an acrylic acid group H₂C=C(-COOR¹⁰)-, in which R¹⁰ is H, C₁-C₃ alkyl, 1,6-dimethylphenyl or mesityl, when Z = CO-NR⁶: a vinyl group H₂C=C(-R⁹)-, in which R⁹ is H or CH₃,
m = 1 or 2,
n = 1 or 2,
SP = absent or an (n+m)-valent linear or branched aliphatic C₁-C₆ residue, an (n+m)-valent cycloaliphatic C₃-C₁₀ residue or an (n+m)-valent heterocyclic C₃-C₁₀ residue, wherein the residues can be substituted or unsubstituted.

5. Dental material according to one of the preceding claims, in which the SP is substituted by C₁-C₅ alkyl, Cl, Br and/or OH.

6. Dental material according to one of claims 1 to 5, in which the filler is an amorphous filler based on one or more metal oxides and/or silicon oxide.

7. Dental material according to claim 6, in which the filler is based on ZrO₂, Ta₂O₃, TiO₂, a mixed oxide of SiO₂, ZrO₂ and/or TiO₂, Yb₂O₃, Y₂O₃; YbF₃, Al₂O₃ and AIO(OH) Böhmite, pyrogenic silicic acid or precipitated silicic acid.

8. Dental material according to one of claims 1 to 5, in which the filler is based on powdered quartz, powdered glass ceramic or powdered glass.

9. Dental material according to one of claims 1 to 5, in which the filler is based on an X-ray opaque metallic compound such as ytterbium trifluoride.

10. Dental material according to one of the preceding claims which comprises
(a) 5 to 90 wt % filler that is surface-modified with a compound of Formula (I),
(b) 9.9 to 90 wt % of a radically polymerisable monomer,
(c) 0.1 to 5.0 wt % of initiator for the radical polymerisation, and
(d) up to 70 wt % solvent,
each based on the total weight of the dental material.

11. Dental material according to one of the preceding claims which comprises a diluent monomer that is selected from mesityl methacrylate, *N*-ethylacrylamide, *N,N*-dimethacrylamide, *N*-(2-hydroxyethyl)acrylamide, *N*-methyl-*N*-(2-hydroxyethyl)acrylamide, *N-*ethylmethacrylamide and *N*-(2-hydroxyethyl)methacrylamide, and/or a crosslinking monomer that is selected from 1,6-bis(3-vinyl-2-pyrrolidonyl)-hexane, methylene- and ethylenebisacrylamide, *N,N*'-diethyl-1,3-bis(acrylamido)-propane, 1,3-bis(methacrylamido)-propane, 1,4-bis(methacrylamido)-butane, 1,4-bis(acrylamido)-butane and 1,4-bis(acrylamido)-piperazine.

## Revendications

1. Matériau de dentisterie, **caractérisé en ce qu'**il contient :
a) une charge, modifiée en surface avec un composé de formule (I) :
[(PG)-R¹-Z]ₙ-SP-[Y-R²-(AG)]ₘ (I)
dans laquelle
- AG représente un groupe de formule -COOH, -P(=O)(OH)₂, -O-P(=O)(OH)₂ ou -SO₂OH ou un groupe chélateur,
- R¹ représente un groupe alcanediyle en C₁-C₃ ou cyclopropanediyle, ou ne représente rien,
- R² représente un groupe alcanediyle en C₁-C₁₀, ou ne représente rien,
- Y représente un ou des chaînon(s) symbolisé(s) par O, S, CO-NH, O-CO-NH ou NH-CO-NH, ou ne représente rien, étant entendu que Y ne représente rien quand R² ne représente rien,
- Z ne représente rien ou représente un ou des chaînon(s) symbolisé(s) par O, S, CO-NR⁶, O-CO-NH ou NH-CO-NH, où R⁶ représente un atome d'hydrogène ou un groupe alkyle en C₁-C₆,
- PG représente un groupe polymérisable par voie radicalaire, de formule 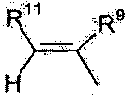 dans laquelle
- R⁹ représente un atome d'hydrogène, un groupe alkyle en C₁-C₃ ou hydroxy-alkyle en C₁-C₃, ou un groupe symbolisé par COOR¹⁰, où R¹⁰ représente un atome d'hydrogène, un groupe alkyle en C₁-C₁₀, ou un groupe 1,6-diméthyl-phényle ou mésityle,
- et R¹¹ représente un atome d'hydrogène ou un groupe phényle,
- l'indice m vaut 1 ou 2,
- l'indice n vaut 1, 2, 3 ou 4,
- et SP ne représente rien ou représente un groupe aliphatique en C₁-C₃₀, linéaire ou ramifié, de valence n+m, dont la chaîne d'atomes de carbone peut être interrompue par un ou des chaînon(s) symbolisé(s) par O, S, CO-NH, O-CO-NH ou NH-CO-NH, un groupe aromatique en C₆-C₁₈, de valence n+m, un groupe cycloaliphatique en C₃-C₁₈, de valence n+m, ou un groupe hétérocyclique en C₃-C₁₈, de valence n+m, lesquels groupes peuvent ne porter aucun substituant ou porter un ou des substituant(s) alkyle en C₁-C₅, chloro, bromo et/ou hydroxy ;
b) un liant polymérisable par voie radicalaire, qui contient :
- en option, au moins un monomère diluant et stable vis-à-vis d'une hydrolyse, qui est choisi parmi les mono-(méth)acrylates, acrylamides N-monosubstitués ou N,N-disubstitués, méthacrylamides N-monosubstitués et N-vinyl-pyrrolidones,
- au moins un monomère réticulant et stable vis-à-vis d'une hydrolyse, qui est choisi parmi les pyrrolidones réticulantes, bis-acrylamides et bis-(méth)acrylamides,
- et au moins un monomère acide comportant au moins un groupe acide,
étant entendu qu'il faut comprendre, par « monomère stable vis-à-vis d'une hydrolyse », un monomère qui, laissé dans de l'eau, ou dans un mélange d'eau et de solvant miscible à l'eau, en une concentration de 20 % en poids, à un pH de 2,0 et à 37 °C, n'est hydrolysé qu'à un degré de moins de 5 % au bout de six semaines,
et étant entendu que le monomère acide est choisi parmi les composés suivants : 2-[4-(dihydroxy-phosphoryl)-2-oxa-butyl]-acrylate d'éthyle, acide 2-[4-(dihydroxy-phosphoryl)-2-oxa-butyl]-acrylique, 2-[4-(dihydroxy-phosphoryl)-2-oxa-butyl]-acrylate de 2,4,6-triméthyl-phényle, dihydrogéno-phosphate de 6-(N-acryloyl-amino)-hexyle, dihydrogéno-phosphate de 6-(N-méthacryloyl-amino)-hexyle, dihydrogéno-phosphate de 1,3-bis-(N-acryloyl-amino)-prop-2-yle, et dihydrogéno-phosphate de 1,3-bis-(N-méthacryloyl-amino)-prop-2-yle ;
c) un amorceur pour polymérisation par voie radicalaire ;
d) et de l'acétone, de l'iopropanol et/ou de l'éthanol.

2. Matériau de dentisterie conforme à la revendication 1, dans lequel le groupe chélateur est un groupe de type β-dicétone, de formule -R⁷-C(=O)-CH₂-C(=O)-R⁸ où R⁷ représente un groupe alcanediyle en C₁-C₃ et R⁸ représente un groupe alkyle en C₁-C₃, ou de type acide salicylique, aldéhyde salicylique, glycinate, phénanthroline, bipyridyle, éthylènediamine, diéthylènetriamine, imino-diacétate, tris-(2-amino-éthyl)-amine, amino-triacétate ou éthylènediamine-tétraacétate.

3. Matériau de dentisterie conforme à l'une des revendications précédentes, dans lequel PG représente un groupe de type vinyle, de formule H₂C=C(-R⁹)-, un groupe de type acide acrylique, de formule H₂C=C(-CO-OR¹⁰)-, un groupe allyle, un groupe styryle et/ou un groupe vinyl-cyclopropyle.

4. Matériau de dentisterie conforme à l'une des revendications précédentes, dans lequel au moins l'un des symboles de la formule (I) a l'une des significations suivantes :
- AG représente un groupe de formule -P(=O)(OH)₂, -O-P(=O)(OH)₂ ou -COOH,
- R¹ représente un groupe méthanediyle ou cyclopropanediyle, ou ne représente rien,
- R² représente un groupe alcanediyle en C₁-C₃, ou ne représente rien,
- Z représente des chaînons symbolisés par CO-NR⁶ ou ne représente rien,
- Y ne représente rien,
- PG représente, si Z ne représente rien, un groupe de type vinyle, de formule H₂C=C(-R⁹)- où R⁹ représente un atome d'hydrogène ou un groupe méthyle, éthyle, hydroxy-méthyle ou hydroxy-éthyle, ou bien un groupe de type acide acrylique, de formule H₂C=C(-COOR¹⁰)-où R¹⁰ représente un atome d'hydrogène, un groupe alkyle en C₁-C₃, ou un groupe 1,6-diméthyl-phényle ou mésityle,
et si Z représente des chaînons symbolisés par CO-NR⁶, un groupe de type vinyle, de formule H₂C=C(-R⁹)- où R⁹ représente un atome d'hydrogène ou un groupe méthyle,
- l'indice m vaut 1 ou 2,
- l'indice n vaut 1 ou 2,
- et SP ne représente rien ou représente un groupe aliphatique en C₁-C₆, linéaire ou ramifié, de valence n+m, un groupe aromatique en C₆-C₁₀, de valence n+m, un groupe cycloaliphatique en C₃-C₁₀, de valence n+m, ou un groupe hétérocyclique en C₃-C₁₀, de valence n+m, lesquels groupes peuvent ne porter aucun substituant ou porter un ou des substituant(s).

5. Matériau de dentisterie conforme à l'une des revendications précédentes, dans lequel le groupe représenté par SP porte un ou des substituant(s) alkyle en C₁-C₅, chloro, bromo et/ou hydroxy.

6. Matériau de dentisterie conforme à l'une des revendications 1 à 5, dans lequel la charge est une charge amorphe à base d'un ou de plusieurs oxyde(s) de métal ou de métaux et/ou d'oxyde de silicium.

7. Matériau de dentisterie conforme à la revendication 6, dans lequel la charge est à base de ZrO₂, Ta₂O₃, TiO₂, d'un oxyde mixte de SiO₂, ZrO₂ et/ou TiO₂, d'oxyde Yb₂O₃, Y₂O₃ ou Al₂O₃, de YbF₃, de böhmite AlO(OH), d'acide silicique pyrogéné ou d'acide silicique précipité.

8. Matériau de dentisterie conforme à l'une des revendications 1 à 5, dans lequel la charge est à base d'une poudre de quartz, de vitrocéramique ou de verre.

9. Matériau de dentisterie conforme à l'une des revendications 1 à 5, dans lequel la charge est à base d'un composé métallique opaque aux rayons X, tel le trifluorure d'ytterbium.

10. Matériau de dentisterie conforme à l'une des revendications précédentes, qui contient :
a) de 5 à 90 % en poids de charge modifiée en surface avec un composé de formule (I),
d) de 9,9 à 90 % en poids de monomères polymérisables par voie radicalaire,
c) de 0,1 à 5,0 % en poids d'amorceur pour polymérisation par voie radicalaire,
d) et jusqu'à 70 % en poids de solvant,
étant entendu que chacun de ces pourcentages est rapporté à la masse totale du matériau de dentisterie.

11. Matériau de dentisterie conforme à l'une des revendications précédentes, qui contient
- un monomère diluant, qui est choisi parmi les méthacrylate de mésityle, N-éthyl-acrylamide, N,N-diméthyl-acrylamide, N-(2-hydroxy-éthyl)-acrylamide, N-méthyl-N-(2-hydroxy-éthyl)-acrylamide, N-éthyl-méthacrylamide et N-(2-hydroxy-éthyl)-méthacrylamide,
- et/ou un monomère réticulant, qui est choisi parmi les 1,6-bis-(3-vinyl-2-pyrrolidonyl)-hexane, méthylène-bis(acrylamide), éthylène-bis(acrylamide), N,N'-diéthyl-1,3-bis(acrylamido)-propane, 1,3-bis(méthacrylamido)-propane, 1,4-bis(méthacrylamido)-butane, 1,4-bis(acrylamido)-butane et 1,4-bis(acrylamido)-pipérazine.
